# EUROPEAN PATENT APPLICATION

(11) **EP 4 792 829 A1**
(43) Date of publication of application: **19.08.2026**
(21) Application number: 25158639.2
(22) Date of filing: 18.02.2025
(51) Int. Cl.: A61K 39/00, A61K 48/00, C12N 15/86

(54) **VIRUS-BASED EXPRESSION OF PROTEINS AND GENES**

(71) Applicant: Sagitta Biotech, 4031 Liège (BE)
(72) Inventor: DAMIEN, Benjamin, 4031 Liège (BE); GAZON, Hélène, 4031 Liège (BE)
(74) Representative: Calysta NV

(57) **Abstract**

A nucleic acid molecule in the form of a plasmid comprising an origin of replication, a promoter for the transcription into viral RNA; a 3' NCT triplet upstream of regions for the insertion of gene(s) of interest, separated by Measeles virus intergenic regions, and one 5' NCT sharing at least 95% of identity with SEQ ID NO: 11 over its full-length, wherein the 3' NCT triplet shares at least 95% of identity with SEQ ID NO:14 over its full-length, the corresponding helper nucleic acid molecules, a process for the production of virosomes based on these nucleic acid based molecules, the obtained virosomes and their therapeutical uses.

## Description

### Technical Field

The present invention relates to the production of virosomes from Measle virus (MV) for a safe administration of proteins to a patient, for instance to induce a protective immune response against a pathogen, using virosomes expressing antigens of this pathogen. The proteins are potentially expressed on the surface of the virosome and also encoded on a RNA contained in the virosome and delivered in the target cells.

### Prior art

Reverse genetics is a molecular biology method for creating a new virus from its cloned cDNA. This technique facilitates the understanding of viral biology and encourages the further development of novel vaccines and vectors for gene delivery. The first recovery of a non-segmented negative-strand RNA virus entirely from cloned cDNA was achieved in 1994 for the rabies virus. Since then, other non-segmented negative-strand RNA viruses have been recovered from cloned cDNA

The use of attenuated, inactivated or inactive virus for vaccination or gene transfer purposes is known.

Measles Virus is one of them, yet not used in clinical practice. One possible explanation is associated with the natural prevalence of MV-antibodies in Human populations; hence such vector will be rapidly neutralized by the immune system. Moreover, this virus cycle and/or mode of action is very complex, hindering its industrial uses for *in vitro* purposes and/or for clinical developments such as the development of vaccines. The virus comprises 6 important genes, encoding N (nucleocapsid), P (phosphoprotein), M (matrix protein), F (fusion protein), H (hemagglutinin) and L (RNA-dependent RNA polymerase). The first attempt of reverse genetics system was based on the co-transfection of two plasmids (Radecke et al, 1995). Several more recent attempts have been performed. However, those methods remained inefficient. Among them, several attempts were made to increase the efficiency of the so-called MV rescue (artificial reproduction of the viral cycle so as to use it for research or medical purposes). These include modifying the use of other susceptible cells (CHO-hSLAM) by Schneider (1997), following the use of recombinant modified vaccinia virus Ankara (MAV-T7) (Kovacs 2003) or vaccinia virus Lister vaccine strain (Nakatsu 2006) to increase sufficient T7 RNA polymerase expression, or involving the use of host polymerase II promoters (CMV) to drive viral RNA synthesis (Martin 2006, Li 2011). However, the efficiency of the recovery remained relatively low, and there is a need for improvement. Rescue efficiencies in the systems with T7-expressing vaccinia virus are high, though laborious cleaning steps are required.

For instance, WO 2016/110869 describes the use of MV sequences, a construct to allow the production of one single antigen of interest by a patient with (i) one cloning plasmid comprising the entire anti-genome of measles virus, or MV genome like replicon RNA coding for non-MV genes along with a subset of MV genes, (ii) one helper plasmid coding for and expressing N, P, L proteins respectively.

However, to this day, no vaccines using a rescued viral genome has been successfully developed.

### Brief summary of the invention

It is the object of the invention to provide a nucleic acid molecule (DNA) in the form of a plasmid comprising:
- a promoter for the transcription into viral RNA;
- a 3'NCT region upstream of
- 1, 2 or more regions for the insertion of gene(s) of interest, separated by intergenic regions, or
   1,2 or more cassette(s) for the expression of a protein of interest, and
- one 5'NCT sharing at least 95% of identity with SEQ ID NO: 11 over its full-length,
wherein
the 3'NCT region shares at least 95% of identity with SEQ ID NO:14 over its full-length, and
the intergene regions share at least 95% of identity with SEQ ID NO:8, SEQ ID NO:9, SEQ ID NO:10, SEQ ID NO:18 and/or SEQ ID NO:19 over their full-length,
the said nucleic acid molecule being under the control of a RNA Polymerase I/II and/or comprises the T7 promoter (SEQ ID NO:17) to allow the transcription of the DNA into a single stranded RNA and under the control of the MV RNA-dependent RNA polymerase to replicate the translated RNA and produce a mRNA from the RNA template,
the said cassette(s) for the expression of a protein of interest or the said regions for the insertion of genes, preferably after the insertion of the said genes, having a combined size being a multiple of 6 base pairs, as calculated starting from the first nucleotide of the SEQ ID NO:14 to the last nucleotide of SEQ ID NO:11.

Preferably, this nucleic acid molecule is further comprising an hairpin region upstream of the 3'NCT regio, sharing preferably at least 95% of identity with SEQ ID NO:15 or SEQ ID NO:21, and/or an excision region downstream the 5'NCT, sharing preferably at least 95% of identity with SEQ ID NO:16.

Preferably, this nucleic acid molecule is further comprising an expression cassette for the M protein, the said M protein sharing at least 95% of identity with SEQ ID NO 13, preferably the said cassette being placed after an intergene region and just upstream of SEQ ID NO:11.

A related and complementary aspect of the present invention is a nucleic acid molecule in the form of a plasmid L comprising
- a promoter for the transcription into RNA
- a cassette encoding L protein of MV, the said L protein sharing at least 95% of identity with SEQ ID NO:6,
and a poly A signal.

Another related and complementary aspect of the present invention relates to a nucleic acid molecule in the form of a plasmid NP comprising
- a promoter for the transcription into RNA, and
- genetic element for the expression of the protein N, the said protein N sharing at least 95% of identity with SEQ ID NO:4 over its full-length, and genetic element for the expression of the protein P, the said protein P sharing at least 95% of identity with SEQ ID NO:2 over its full-length, wherein

either the genetic elements for the expression of the protein N and the genetic elements for the expression of the protein P are separated by a cleaving site so as to generate two messengers RNA, or wherein
the said plasmid comprises the genetic elements for the expression of the protein N followed by a poly-adenylation signal and a second promotor for the transcription into RNA of the genetic elements for the expression of the protein P.

Preferably, the RNA polymerase II promoter is pActb, pCMV, pCMV+intron, pEF1A, pEFS, pCAG, pCBh, pSFFV, pSV40, phBoV1 or phPGK, pUBC, and/or, the RNA polymerase II promoter is shorter than 1000 nucleotides and/or wherein the plasmids are shorter than 10000 base pairs.

Preferably, the nucleic acid construct(s) of the present invention is (are) further comprising a cassette for eukaryotic and/or prokaryotic selection.

Another related aspect of the present invention relates to a genetic system comprising the two nucleic acid molecules L and NP described here above, preferably wherein the RNA polymerase promoter of the nucleic acid molecule L is different of the RNA polymerase promoter of the nucleic acid molecule NP.

In such a genetic system, wherein the nucleic acid molecule L and NP both comprise a cassette for eukaryotic and/or prokaryotic selection, preferably, the said cassette for eukaryotic and/or prokaryotic selection of the nucleic acid molecule L is different as compared with the said cassette for eukaryotic and/or prokaryotic selection of the nucleic acid molecule NP.

Another related aspect of the present invention relates to a process for the production of virosomes expressing one or several protein(s) of interest comprising the steps of
- growing a eukaryotic cell line, preferably a mammalian cell line in a cell culture medium;
- engineering the first nucleic acid molecule (comprising the 3'NCT region, gene(s) of interest, intergene, 5'NCT) and
- transfecting the nucleic acid molecule within the said selected eukaryotic cells and
- recovering the said virosomes in the cell culture medium.

Preferably, this process is further comprising the step of engineering the genetic system described here above and co-transfecting the three different nucleic acid molecules within the said eukaryotic cells.

Preferably, the recovering of the virosomes comprises a DNAse treatment to hydrolyze host cell's DNA, filtration and/or precipitation of the virosome.

Another related aspect of the present invention relates to the virosomes obtainable by this process.

Another related aspect of the present invention is the Measles virus-derived virosomes comprising one, two or more protein(s) of interest, and a RNA sequence comprising;
- a 3'NCT region upstream of
- 1,2 or more cassette(s) for the expression of this (these) protein(s) of interest, each cassette being separated by a different intergene region, and
- a 5' NCT region sharing at least 95% of identity with SEQ ID NO 11 over its full-length,
- preferably a cassette for the expression of the M protein, the said M protein sharing at least 95% of identity with SEQ ID NO 13, preferably the said cassette being placed after an intergene region and just upstream of SEQ ID NO:11,

wherein the 3' NCT region shares at least 95% of identity with SEQ ID NO:14 over its full-length, and
the intergene regions share at least 95% of identity with SEQ ID NO:8, SEQ ID NO:9, SEQ ID NO:10, SEQ ID NO:18 and/or SEQ ID NO:19;
the said RNA sequence having a total size being a multiple of 6 nucleotides, as calculated starting from the first nucleotide of the SEQ ID NO:14 to the last nucleotide of SEQ ID NO:11.

Another related aspect of the present invention is a pharmaceutical composition comprising these virosomes, being preferably for vaccination or for gene therapy.

Other embodiments according to the present invention are mentioned in the appended claims and the subsequent description of an embodiment of the invention.

### Brief description of the Drawings

Figure 1 is a FACS analysis of transfection using mCherry virosomes as in the prior art (left panel), or as in the prior art, but refining the culture and transfection protocols (right panel).
Figure 2 shows the structure of the cloning plasmid according to the present invention.

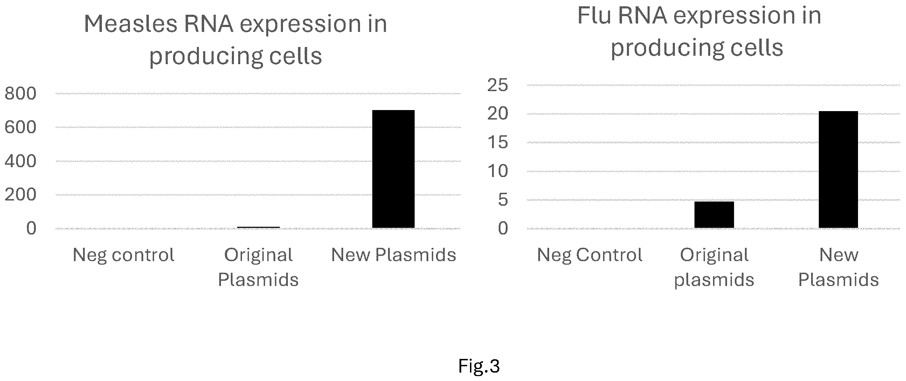

Figure 3 is a bar graph of measles RNA expression in producing cells
Figure 4 shows the antibody induction.
Figure 5 shows the T Cell response using the prior art's protocols (lefts panel; light symbols, untreated; dark symbols, treated) or the constructs according to the present invention.

### Detailed description of an embodiment of the invention

The inventors have firstly attempted to take advantage of the constructs of WO 2016/110869 (prior art).

However, the first experiments have allowed only 0.1% of transfected cells.

Even after several rounds of fine-tuning, they obtained only about 7% of cells expressing the protein of interest.

Moreover, the antigen level was very low, pointing to a low DNA to RNA transcription efficiency, leading to a low translation level.

A low amount of transfected cells combined with a low level of expression resulted in unacceptable performances during *in vitro* and *in vivo* testing, including undetectable immune response in vivo.

In completely redesigning the constructs, with two helper plasmids, the inventors have succeeded in expressing 6 different proteins using one cloning plasmid.

Indeed, the inventors have preferred the complexity of a system with three plasmids to be co-transfected in one cell and have succeeded in obtaining high and consistent production of antigens/proteins of interests. In this system, the two helper plasmids comprise expression cassettes under the control of the cellular RNA polymerase (I or II) and/or under the control of bacterial polymerase, to express the measles' proteins required for the viral cycle (L, N and P), whereas the coding plasmid comprises one expression cassette that will be transcribed by a cellular RNA polymerase (1 or 2) and/or a procaryotic T7, into a single-stranded positive RNA, which will be subsequently processed by the MV proteins to create a negative-stranded RNA so as to create a mRNA encoding the proteins of interest.

Hence, a first aspect of the present invention relates to a nucleic acid molecule in the form of a plasmid (cloning vector) comprising :
- an origin of replication,
- a (dual) promoter (Cellular Polymerase I/II and/or T7 procaryotic polymerase) for the transcription into viral RNA;
- a 3'NCT upstream of
- 1, 2 or more regions for the insertion of gene(s) of interest, separated by intergenic regions, or 1,2 or more cassette(s) for the expression of a protein of interest, and
- one terminator region (5'NCT) sharing at least 95% (preferably at least 97, 98, 99 or even 100%) of identity with SEQ ID NO 11 over its full-length,

wherein the 3'NCT triplet shares at least 95% (preferably at least 97, 98, 99 or even 100%) of identity with SEQ ID NO:14 over its full-length, and the intergene regions share at least 95% (preferably at least 97, 98, 99 or even 100%) with SEQ ID NO:8, SEQ ID NO:9, SEQ ID NO:10, SEQ ID NO:18 and/or SEQ ID NO:19; SEQ ID NO:19 inducing a lower expression,
the said nucleic acid molecule being under the control of a RNA Polymerase (RNA polymerase I/II and/or the T7) promoter to allow the transcription of the DNA into a single stranded RNA and under the control of the MV RNA-dependent RNA polymerase (L protein; see below) to replicate the translated RNA and produce a mRNA from the RNA template,
the said cassette(s) for the expression of a protein of interest or the said regions for the insertion of genes, preferably after the insertion of the said genes, having a combined size being a multiple of 6 base pairs, as calculated starting from the first nucleotide of the SEQ ID NO:14 to the last nucleotide of SEQ ID NO:11.

In other words, even if the construct comprises the region for the insertion of the gene(s) of interest, the region starting at the first nucleotide of SEQ ID NO:14 and finishing at the last nucleotide of SEQ ID NO:11 is at least a multiple of 3 base pairs, allowing an easy insertion of coding sequences.

A related aspect is thus a process to generate such cloning vector with one or several gene(s) of interest, comprising the step of selecting the above vector with the regions for the insertion of one or several gene(s) of interest, of inserting this or these gene(s) of interest and ensuring a multiple of 6 base pairs for the region starting at the first nucleotide of SEQ ID NO:14 and finishing at the last nucleotide of SEQ ID NO:11.

For the construct with the coding sequence(s), one skilled practitioner will easily adapt the size of the region starting at the first nucleotide of SEQ ID NO:14 and finishing at the last nucleotide of SEQ ID NO:11 to match the requirement for the multiple of 6 base pairs, for instance upon addition or removal of one triplet, if needed, while keeping the desired functions of the encoded proteins.

Indeed, the skilled practitioner, possibly using bioinformatic tools, can readily decide a convenient locus for addition or removal of one triplet, and/or the nucleotides forming such triplet to add. The skilled practitioner can also test the functionality of the resulting construct.

In the context of the present invention, preferably, the sequence of identity between nucleotides sequences (e.g. at least 95%) is calculated upon a BLASTn alignment of the complete sequence listed in the present patent application and the sequence to be compared.

Conversely, when the cassettes defined here below are for the translation of proteins, the identity is preferably determined using BLASTp with the default parameter (BLOSUM 62 matrix gap creation; cost 11, extension cost 1; word size of 5). The full-length of the defined peptide (protein) sequence is aligned by the BLASTp algorithm with the protein to test. In these conditions any identical residue (and also possibly labelled as conserved (+)) is considered. Hence in these conditions, the proteins share at least 95% of identical (or conserved) aminoacids.

The inventors have considered several options for the promoter:
- RNA polymerase I;
- RNA polymerase II
- The bacterial T7.

The RNA polymerase I offers the advantage of allowing the transcription of long fragments and the disadvantage of requiring a nuclear localization of the plasmid.

On the other hand, the bacterial T7 promoter allows high levels of transcription and a cytoplasmic action, but needs a cell line expressing the bacterial T7 polymerase.

Preferably, if a T7 promoter is inserted upstream, a T7 terminator is inserted downstream of the 5'NCT region.

Hence the combination of one nuclear promoter (e.g. the Polymerase I) with the T7 allows a more robust expression.

The inventors have found that the potential disadvantage associated to the use of the T7 promoter is also rescued upon placing the cassette for the expression of the M (see below) protein downstream this RNA molecule: with this construct, only full-length transcripts can lead to virosomes.

Other promoters for the RNA Polymerase II are known, for instance those listed herebelow:

| ***Name*** | **Description** | **Notes** |
|---|---|---|
| *pActb* | Human Actin B promoter | |
| *pCMV* | Human cytomegalovirus immediate early enhancer/promoter | Strong promoter |
| *pCMV+intron* | Human cytomegalovirus immediate early enhancer/promoter fused with the splicing signal from the human beta-globin intron 2 | Strong promoter |
| *pEF1A* | Human eukaryotic translation elongation factor 1 α1 promoter | Strong promoter |
| *pEFS* | Human eukaryotic translation elongation factor 1 α1 short form | Medium-strength promoter |
| *pCAG* | CMV early enhancer fused to modified chicken β-actin promoter | Strong promoter |
| *pCBh* | CMV early enhancer fused to modified chicken β-actin promoter | Strong promoter |
| *pSFFV* | Spleen focus-forming virus promoter | Strong promoter |
| *pSV40* | Simian virus 40 enhancer/early promoter | Moderate to strong expression |
| *phBoV1* | human bocavirus type 1 promoter | Strong promoter |
| *phPGK* | Human phosphoglycerate kinase 1 promoter | For low to moderate expresseion |
| *pUBC* | Human ubiquitin C promoter | Weak promoter |

The presence of intergene regions is essential to allow the expression of the different genes to form different proteins (proteins of interest and, preferably, the viral M protein). One skilled practitioner can select which intergene region to place upstream which cassette so as to fine tune the relative expression of the different proteins. Possibly an intergene region is placed only once in the construct, even if the same intergene region can be placed twice in the construct. The organization of the intergene regions in the construct is of interest in vaccination, for instance to control the humoral versus cellular response, but also in gene therapy, with the expression of exposed proteins at a desired level (for instance to target one cell type or one tissue) and the expression of the curative proteins (or genes) at another level and also in the general design with one level of expression of the M protein (if present) and another level of expression of the protein(s) of interests.

Hence, preferably, this nucleic acid molecule is further comprising an expression cassette for the M protein, the said M protein sharing at least 95% (preferably at least 97, 98, 99 or even 100%) of identity with SEQ ID NO 13, preferably the said cassette being placed after an intergene region and just upstream of SEQ ID NO:11.

The inventors do consider that the expression of the M protein by the transfected cell is important for the budding of the viruses.

The inventors have advantageously placed the sequence encoding the M protein on the cloning plasmid, after having determined that this design ensures the production of complete coding virosomes (especially when a T7 promoter is present on this construct). Moreover, the cassette of the M protein has been advantageously placed at the distal extremity of the promoter for the same purpose.

More preferably a Poly adenylation sequence is inserted downstream of the 5'NCT region or (if present), downstream of the T7 terminator.

Preferably, this DNA molecule is further comprising hairpin-forming regions (just) upstream of the 3'NCT region, such as SEQ ID NO:15 or SEQ ID NO:21 (or genetic elements allowing a similar effect for the transcription, preferably sharing motives with SEQ ID NO:15 and/or SEQ ID NO:21) and/or excision region(s) (just) downstream the 5'NCT region, being preferably SEQ ID NO:16.

In other words, in this region, upstream of the viral genome (and the embedded sequences coding for the gene(s) of interest), there is advantageously a genetic element helping the transcription, preferably a sequence forming an hairpin, such as SEQ ID NO:15 or SEQ ID NO:21.

Conversely, in this region, downstream of the viral genome, there is advantageously a sequence coding for an excision element precisely at the end of SEQ ID NO:15, such as a ribozyme, for instance SEQ ID NO:16.

This allows the production of the coding RNA by the polymerase L, regardless the associated sequences potentially present, especially when several promoters are placed upstream of the coding sequence and/or terminator, poly A region and one or several selection cassettes are placed downstream the coding sequence.

A corresponding and related aspect of the present invention is a nucleic acid molecule in the form of a plasmid L comprising :
- an origin of replication
- a promoter for the transcription into RNA
- a cassette encoding L protein of MV, the said L protein sharing at least 95% (preferably at least 97, 98, 99 or even 100%) of identity with SEQ ID NO:6,
and a poly A signal.

This is a part of the "helper system".

The inventors have chosen to generate a plasmid encoding only the L protein since its large size and despite expected difficulties caused by its separation from the N and P expression cassettes. The inventors have, instead, found a better control of the expression of this protein, when carried on a specific plasmid.

Another corresponding and related aspect of the present invention is a nucleic acid molecule in the form of a plasmid NP comprising
- an origin of replication,
- a promoter for the transcription into RNA, and
- genetic element for the expression of the protein N, the said protein N sharing at least 95% (preferably at least 97, 98, 99 or even 100%) of identity with SEQ ID NO:4 over its full-length, and genetic element for the expression of the protein P, the said protein P sharing at least 95% (preferably at least 97, 98, 99 or even 100%) of identity with SEQ ID NO:2 over its full-length, wherein either

the genetic elements for the expression of the protein N and the genetic elements for the expression of the protein P are separated by a cleaving site so as to generate two messengers RNA, or wherein
the said plasmid comprises the genetic elements for the expression of the protein N followed by a poly-adenylation signal and a second promotor for the transcription into RNA of the genetic elements for the expression of the protein P.

A preferred cleaving site is P2A or T2A; exemplary sequences share at least 95% (preferably at least 97, 98, 99 or even 100%) of identity with SEQ ID NO:7 or SEQ ID NO:20.

This is the second part of the "helper system".

The inventors have found that the dissociation of the helper system in two distinct molecules allows a more predictable and robust expression.

The risk of missing one of the three plasmids has been advantageously mitigated upon placing the cassette for the M protein on the cloning plasmid (see above).

Preferred promoters of these nucleic acid molecules are RNA polymerase II promoter, such as those chosen in the group consisting of pActb, pCMV, pCMV+intron, pEF1A, pEFS, pCAG, pCBh, pSFFV, pSV40, phBoV1 or phPGK, pUBC.

Alternatively, or in addition, a preferred promoter is the T7 (SEQ ID NO:17).

Alternatively, or in addition, a preferred promoter is a RNA polymerase I promoter.

Advantageously, the RNA polymerase promoters are shorter than 2000 base pairs, preferably shorter than 1500 base pairs, more preferably shorter than 1000, 900, 800, 700 or even 600 base pairs.

This avoids too large plasmid constructs, even if the incorporation of the polymerase (I or II) promoters are much larger than the T7 promoter; in other words, the incorporation of the polymerase (I or II) promoters in the plasmids is against a prejudice since the large size of the plasmids is a problem.

Preferably, these nucleic acid molecules (especially for the "helper system") further comprising a cassette for eukaryotic and/or prokaryotic selection.

A related aspect of the present invention is a genetic ("helper") system comprising the two nucleic acid molecules L and NP described above, wherein preferably the RNA polymerase promoter of the nucleic acid molecule L is different of the RNA polymerase promoter of the nucleic acid molecule NP.

This allows to better tune the relative expression levels of the different proteins, where the inventors have found that the expression level of the L protein can be much lower.

Moreover, this allows avoiding the saturation of one single promoter.

Conversely, or in addition, a (the) genetic system comprising the nucleic acids molecule L and NP both comprise a cassette for eukaryotic and/or prokaryotic selection, wherein the said cassette for eukaryotic and/or prokaryotic selection of the nucleic acid molecule L is different as compared with the said cassette for eukaryotic and/or prokaryotic selection of the nucleic acid molecule NP.

This allows a specific selection, ensuring the presence of the two plasmids.

A related aspect of the present invention is a process for the production of virosomes expressing one or several protein(s) of interest comprising the steps of :
- growing a eukaryotic cell line, preferably a mammalian cell line in a cell culture medium;
- engineering the cloning vector as described here above and
- transfecting the nucleic acid molecule within the said selected eukaryotic cells and
- recovering the said virosomes in the cell culture medium.

Preferably, this process is further comprising the step of engineering the "helper" genetic system described here above (plasmid L and NP) and co-transfecting the three different nucleic acid molecules within the said eukaryotic cells.

The alternative without the helper system or without the full helper system is based on competent cell lines expressing L, N and/or the P proteins at the required levels.

Another related aspect of the present invention relates to the virosomes obtainable by this process.

Conversely, another related aspect of the present invention relates to Measles Virus-derived virosomes comprising one, two or more protein(s) of interest, and a RNA sequence comprising;
- a 3'NCT region upstream of
- 1,2 or more cassette(s) for the expression of a protein of interest being separated by an intergene region, and
- one 5'NCT region sharing at least 95% (preferably at least 97, 98, 99 or even 100%) with SEQ ID NO 11 over its full-length,

wherein the 3'NCT region shares at least 95% (preferably at least 97, 98, 99 or even 100%) of identity with SEQ ID NO:14 over its full-length, and the intergene regions share at least 95% (preferably at least 97, 98, 99 or even 100%) with SEQ ID NO:8, SEQ ID NO:9, SEQ ID NO:10, SEQ ID NO:18 and/or SEQ ID NO:19,
the RNA sequence having a combined size being a multiple of 6 base pairs, as calculated starting from the first nucleotide of the SEQ ID NO:14 to the last nucleotide of SEQ ID NO:11.

Still another related aspect of the present invention relates to a pharmaceutical composition comprising these virosomes.

Such a pharmaceutical composition is advantageously for vaccination (cancer, infectious disease, therapeutic vaccination to correct unwanted immune responses) or for gene therapy.

Indeed, the inventors have found that the technology allows to elicit either a humoral response, a cellular response, or a balance of the two, which provides a significant advantage over mRNA vaccines or over vaccines based on single epitopes.

The virosomes used for these pharmaceutical compositions are advantageously engineered to express at their surface a targeting protein, as well as therapeutic proteins and/or nucleic acid sequences encoding them.

Further characteristics and advantages of the present invention will be derived from the non-limitative following description, and by making reference to the drawings and the examples.

### Examples

### Comparative Example

A MV-based construct as in the prior art, with a coding vector for the expression of a gene of interest and a helper vector encoding N, P and L proteins of MV has been generated for the expression of mCherry protein in Vero cells.

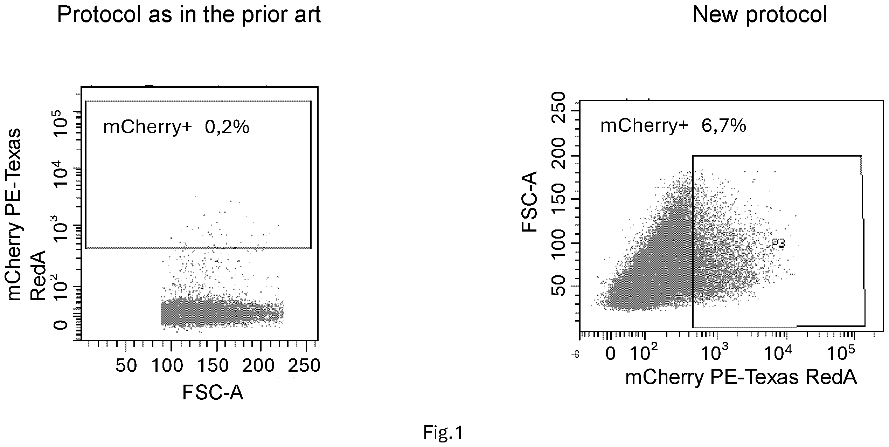

Unfortunately, there was almost no expression (Figure 1, left panel).

The inventors have tried to refine the experimental system, and have invested significant time and energy in adapting the cell type (HEK293 cells), culture medium, the culture conditions and the transfection condition, yet still based on cationic lipids. However, they obtained no significant improvement, only a very marginal expression, as shown in Figure 1, right panel, up to 6.7% of positive cells and a rather low expression level.

This might explain why, in practice, no commercial MV-based system is available at large, commercial or clinical, scale.

### Example 1 design of the new construct

Against this prejudice, the inventors have reconsidered the whole MV-based system. First of all, the inventors have decided to separate the system for the expression of the L protein from the system for the expression of the proteins N and P: instead of one helper vector, the inventors have designed two helper plasmids acting synergically. This approach, although sharing less features with those of the wild-type MV where there is a natural regulation for the correct expression levels of these proteins, is, in practice, unexpectedly more potent.

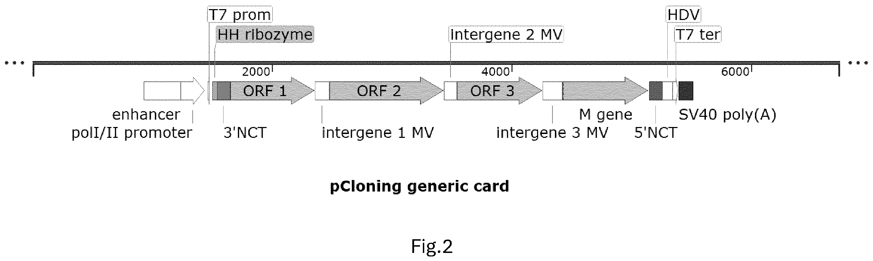

On the other hand the cloning vector has been simplified and focused on the key MV elements identified by the inventors (Figure 2): the MV 3' NCT region, one or several MV intergene regions, a cassette for the expression of the MV M protein (downstream) and the MV 5' NCT region (terminator). Moreover, the inventors have adapted the final constructs (after the incorporation of the gene(s) of interest), so that the length from the first nucleotide of the 3' NCT to the last nucleotide of the 5'NCT is a multiple of 6 base pairs.

The inventors has further completed the design in adding a dual promotor upstream of the 3'NCT, here a Pol 1 promoter and a T7 (the transfected cell line do express the T7 polymerase), separated by a region encoding an hammerhead ribozyme (HH in Figure 2) and, downstream of the 5'NCT, a second ribozyme (sequence from hepatitis delta virus; HDV in Figure 2), a T7 terminator and a poly A region.

### Example 2 - mRNA expression

Thanks to this design with two helper plasmids and the specific cloning plasmid, engineered to comprise two cassettes for expressing two flu proteins (hemagglutinin and neuraminidase), the transfection of HEK293 cells now allows a high expression of MV RNA (encoding the protein M) and of Flu RNA.

### Example 3 - IgG expression

The virosomes have been administered to mice and the level of IgG was measured.

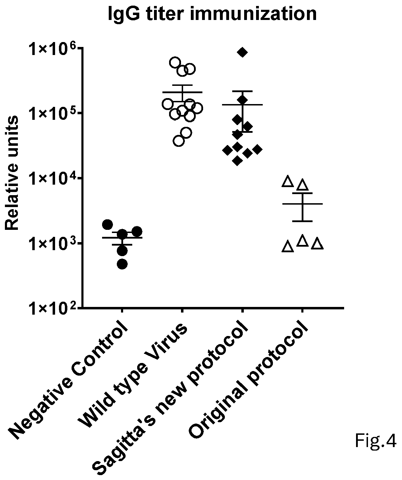

As expected, the WT virus induces a large response (Figure 4).

On the other hand, the protocol for virosome production, based on the prior art but with refined culture conditions (as in Figure 1, right panel), yields only a marginal IgG induction.

The new protocol with two helper plasmids and a redesigned cloning vector allows a very high IgG production.

### Example 4 - T cell activation or IFNg production

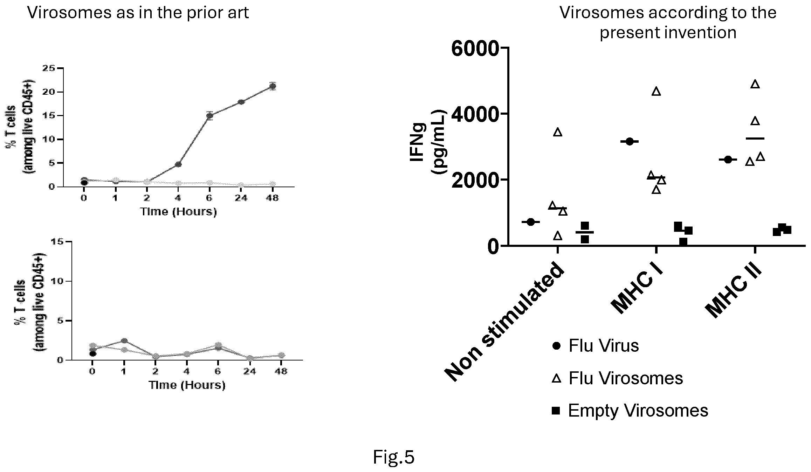

The same system was compared (Figure 5) using the prior art protocol (left panels: upper panel the positive control showing a strong T cell activation and the lower panel being the test with no T cell activation: the treated and untreated lines overlap).

Lymph nodes ganglions have been obtained from mice treated or not with the virosomes (or a direct administration of the two Flu antigens), then challenged with the Flu virus.

Interestingly, in the right panel, the design according to the invention has yielded virosomes expressing Flu proteins allowing a strong activation of both the humoral and the cellular immune response, matching the response obtained by a direct administration of Flu antigens, far above the baseline with empty vectors.

It should be understood that the present invention is not limited to the described embodiments and that variations can be applied without going outside of the scope of the claims.

## Claims

1. A nucleic acid molecule in the form of a plasmid comprising:
- a promoter for the transcription into viral RNA;
- a 3'NCT region upstream of
- 1, 2 or more regions for the insertion of gene(s) of interest, separated by intergenic regions, or
1,2 or more cassette(s) for the expression of a protein of interest, and
- one 5' NCT region sharing at least 95% (preferably at least 97, 98, 99 or even 100%) of identity with SEQ ID NO: 11 over its full-length,
wherein
the 3'NCT region shares at least 95% (preferably at least 97, 98, 99 or even 100%) of identity with SEQ ID NO:14 over its full-length, and
the intergene regions share at least 95% (preferably at least 97, 98, 99 or even 100%) of identity with SEQ ID NO:8, SEQ ID NO:9, SEQ ID NO:10, SEQ ID NO:18 and/or SEQ ID NO:19 over their full-length,
the said nucleic acid molecule being under the control of a RNA Polymerase I/II and/or comprises the T7 promoter (SEQ ID NO:17) to allow the transcription of the DNA into a single stranded RNA and under the control of the MV RNA-dependent RNA polymerase to replicate the translated RNA and produce a mRNA from the RNA template,
the said cassette(s) for the expression of a protein of interest or the said regions for the insertion of genes, preferably after the insertion of the said genes, having a combined size being a multiple of 6 base pairs, as calculated starting from the first nucleotide of the SEQ ID NO:14 to the last nucleotide of SEQ ID NO:11.

2. The nucleic acid molecule of claim 1, further comprising an hairpin region upstream of the 3'NCT region sharing preferably at least 95% of identity with SEQ ID NO:15 or SEQ ID NO:21 and/or an excision region downstream the 5'NCT, sharing preferably at least 95% of identity with SEQ ID NO:16.

3. The nucleic acid molecule of claim 1 or 2 further comprising an expression cassette for the M protein, the said M protein sharing at least 95% of identity with SEQ ID NO 13, preferably the said cassette being placed after an intergene region and just upstream of SEQ ID NO:11.

4. A nucleic acid molecule in the form of a plasmid L comprising
- a promoter for the transcription into RNA
- a cassette encoding L protein of MV, the said L protein sharing at least 95% of identity with SEQ ID NO:6,
and a poly A signal.

5. A nucleic acid molecule in the form of a plasmid NP comprising
- a promoter for the transcription into RNA, and
- genetic element for the expression of the protein N, the said protein N sharing at least 95% of identity with SEQ ID NO:4 over its full-length, and genetic element for the expression of the protein P, the said protein P sharing at least 95% of identity with SEQ ID NO:2 over its full-length, wherein
either the genetic elements for the expression of the protein N and the genetic elements for the expression of the protein P are separated by a cleaving site so as to generate two messengers RNA, or wherein
the said plasmid comprises the genetic elements for the expression of the protein N followed by a poly-adenylation signal and a second promotor for the transcription into RNA of the genetic elements for the expression of the protein P.

6. The nucleic acid molecule of claim 4 or 5 wherein the RNA polymerase II promoter is pActb, pCMV, pCMV+intron, pEF1A, pEFS, pCAG, pCBh, pSFFV, pSV40, phBoV1 or phPGK, pUBC, and/or, wherein the RNA polymerase II promoter is shorter than 1000 nucleotides and/or wherein the plasmids are shorter than 10000 base pairs.

7. The nucleic acid molecule according to any one of the preceding claims 4 to 6 further comprising a cassette for eukaryotic and/or prokaryotic selection.

8. A genetic system comprising the two nucleic acid molecules L and NP according to any one of the preceding claims 4 to 7, preferably wherein the RNA polymerase promoter of the nucleic acid molecule L of claim 4 is different of the RNA polymerase promoter of the nucleic acid molecule NP of claim 5.

9. A genetic system comprising the nucleic acids according to any one of the preceding claims 4 to 7 or the genetic system of claim 8, wherein the nucleic acid molecule L and NP of claims 4 and 5 both comprise a cassette for eukaryotic and/or prokaryotic selection, wherein the said cassette for eukaryotic and/or prokaryotic selection of the nucleic acid molecule L of claim 4 is different as compared with the said cassette for eukaryotic and/or prokaryotic selection of the nucleic acid molecule NP of claim 5.

10. A process for the production of virosomes expressing one or several protein(s) of interest comprising the steps of
- growing a eukaryotic cell line, preferably a mammalian cell line in a cell culture medium;
- engineering the nucleic acid molecule according to any one of claims 1 to 3 and
- transfecting the nucleic acid molecule within the said selected eukaryotic cells and
- recovering the said virosomes in the cell culture medium.

11. The process of claim 10, further comprising the step of engineering the genetic system according to claims 8 or 9 and co-transfecting the three different nucleic acid molecules within the said eukaryotic cells.

12. The process of claims 10 or 11, wherein the recovering of the virosomes comprises a DNAse treatment to hydrolyze host cell's DNA, filtration and/or precipitation of the virosome.

13. The virosomes obtainable by the process of claims to 12.

14. Measles virus-derived virosomes comprising one, two or more protein(s) of interest, and a RNA sequence comprising;
- a 3'NCT region upstream of
- 1,2 or more cassette(s) for the expression of this (these) protein(s) of interest, each cassette being separated by a different intergene region, and
- a 5' NCT region sharing at least 95% of identity with SEQ ID NO 11 over its full-length,
- preferably a cassette for the expression of the M protein, the said M protein sharing at least 95% of identity with SEQ ID NO 13, preferably the said cassette being placed after an intergene region and just upstream of SEQ ID NO:11,
wherein the 3' NCT region shares at least 95% of identity with SEQ ID NO:14 over its full-length, and
the intergene regions share at least 95% of identity with SEQ ID NO:8, SEQ ID NO:9, SEQ ID NO:10, SEQ ID NO:18 and/or SEQ ID NO:19;
the said RNA having a total size being a multiple of 6 nucleotides, as calculated starting from the first nucleotide of the SEQ ID NO:14 to the last nucleotide of SEQ ID NO:11.

15. A pharmaceutical composition comprising the virosomes of claims 13 or 14, being preferably for vaccination or for gene therapy.
